# EUROPEAN PATENT SPECIFICATION

(11) **EP 3 602 008 B1**
(45) Date of publication and mention of the grant of the patent: **29.12.2021**
(21) Application number: 18718578.0
(22) Date of filing: 20.03.2018
(51) Int. Cl.: G01N 21/3504, A61B 5/08, A61M 16/00

(54) **RESPIRATION GAS MONITOR WITH AUTOMATED AND NONOBTRUSIVE FILTER CALIBRATION**
ATEMGASMONITOR MIT AUTOMATISIERTER UND UNAUFFÄLLIGER FILTERKALIBRIERUNG
DISPOSITIF DE SURVEILLANCE DE GAZ DE RESPIRATION À CALIBRAGE DE FILTRE AUTOMATISÉ ET DISCRET

(30) Priority: 20.03.2017 US 201762473514 P
(43) Date of publication of application: 05.02.2020
(73) Proprietor: Koninklijke Philips N.V., 5656 AG Eindhoven (NL)
(72) Inventor: GERETY, Eugene, Peter, 5656 AE Eindhoven (NL)
(74) Representative: Philips Intellectual Property & Standards
(86) International application number: PCT/EP2018/057077
(87) International publication number: WO 2018/172381

(56) References cited:
- EP-A1- 2 065 697
- GB-A- 2 530 095
- US-A- 3 539 804

## Description

### FIELD

The following relates generally to the respiration gas monitor (RGM) device arts, gas detection cell calibration arts, and related arts.

### BACKGROUND

Respiration Gas Monitor (RGM) devices are used for measuring partial pressure or concentration of carbon dioxide (CO₂) in respired air, or some other respired gas such as oxygen (O₂), nitrous oxide (N₂O), or an administered anesthetic gas. An RGM device for measuring CO₂ is commonly referred to as a capnometer. Various gas component detection technologies may be employed. In certain RGM devices employing an optical detector, an infrared light source launches broadband infrared light that passes through a sampling cell through which respired air flows. The opposing optical detector module includes a narrowband filter and an infrared detector. The filter is tuned to pass a wavelength that is strongly absorbed by the target gas, e.g. 4.3 micron for CO₂.

In such a design, the optical detector is calibrated by measuring a reference signal in the absence of the target gas. This entails diverting the respired air flow from the sampling cell, and introducing flow of a reference gas such as air or nitrogen through the sampling cell. The reference gas is chosen to have negligible concentration of the target gas (e.g. negligible CO₂ in the case of a capnometer). Consequently, the measured signal output by the optical detector with the reference gas flowing is the maximum value, as there is negligible absorption by the target gas. With this reference signal determined, the ratio of the signal measured with the infrared light launched through the respired air flow versus the reference signal measured with the infrared light launched through the reference gas flow provides the signal reduction due to infrared absorption by the target gas in the respired air flow.

Document EP 2 065 697 A1 discloses a RGM device and method of operating the same in accord with the preambles of appended claims 1 and 9, respectively.

Document US 3 539 804 A discloses an example of a reference detector and an analytical detector combined in a compensation circuit instead of a single detector together with a filter switching device and numerical compensation.

The following discloses new and improved systems and methods.

### SUMMARY

In one disclosed aspect, a respiration gas monitor (RGM) device comprises a respired air flow path for carrying respired air, an infrared light source arranged to launch infrared light through the respired air flow path, and an optical detector arranged to detect the infrared light after passing through the respired air flow path. An absorption line bandpass filter has a passband that encompasses an absorption line of a target gas. A reference line bandpass filter has a passband over which the respired air is transparent. A control device is operative to switch the RGM device between: a monitoring state in which the absorption line bandpass filter is in the path of the infrared light and the reference line bandpass filter is not in the path of the infrared light; and a calibration state in which the reference line bandpass filter is in the path of the infrared light and the absorption line bandpass filter is not in the path of the infrared light; and characterised by an electro-optical beam steering device operable at: a first electric bias implementing the monitoring state by steering the infrared light to an optical path that passes through the absorption line bandpass filter and does not pass through the reference line bandpass filter, and a second electric bias implementing the calibration state by steering the infrared light to an optical path that passes through the reference line bandpass filter and does not pass through the absorption line bandpass filter.

In another disclosed aspect, a method of operating a respiration gas monitor (RGM) device is disclosed. Respired air is flowed through a respired air flow path. Infrared light is launched through the respired air flow path. While flowing the respired air through the respired air flow path, target gas monitoring is performed, including measuring an infrared transmission signal indicating transmission of the launched infrared light through the respired air flow path with an absorption line bandpass filter disposed in the path of the infrared light and determining a value for the target gas in the respired air from the infrared transmission signal and a reference infrared signal. While flowing the respired air through the respired air flow path, a calibration is performed including measuring the reference infrared signal indicating transmission of the launched infrared light through the respired air flow path with a reference line bandpass filter disposed in the path of the infrared light; characterised by performing the target gas monitoring by operating an electro-optic beam steering device to steer the infrared light to an optical path that passes through the absorption line bandpass filter and does not pass through the reference line bandpass filter; and performing the calibration by operating the electro-optic beam steering device to steer the infrared light to an optical path that passes through the reference line bandpass filter and does not pass through the absorption line bandpass filter.

One advantage resides in providing more accurate monitoring of carbon dioxide or another target gas in respired air.

Another advantage resides in providing more frequent calibration of a respiration gas monitor (RGM) device.

Another advantage resides in providing an RGM device that does not require connection to nitrogen or another calibration gas.

Another advantage resides in providing an RGM device in which the respired gas flow through the RGM device is not interrupted to perform calibration.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention.
FIGURE 1 diagrammatically illustrates a respiration gas monitor (RGM) device.
FIGURES 2-7 diagrammatically illustrate embodiments of a control device of the RGM device of FIGURE 1 operative to switch the RGM device between a monitoring state, in which the target gas is monitored, and a calibration state.
FIGURE 8 diagrammatically shows an operational flow chart for operation of the RGM device of FIGURE 1.

### DETAILED DESCRIPTION

In a respiration gas monitor (RGM) device employing optical detection of the target gas, the infrared light source typically includes an infrared emitting element (e.g. a ceramic element) that is resistively heated by conducting an electrical current pulse train through the infrared element. The infrared element is resistively heated is to a temperature effective to produce broadband blackbody radiation with strong emission in the infrared. This infrared emission is strongly dependent on the precise temperature of the heated infrared element. The temperature of the heated infrared emitting element can drift over time during operation of the RGM device. The infrared light detector may be a lead selenide (PbSe) detector, a microbolometer, thermocouple, pyroelectric detector, or the like, and its sensitivity is also usually strongly temperature dependent. The detector temperature can also drift over time during operation of the RGM device. Depending upon the thermal stabilities of the IR source and sensor, the calibration of the optical detection cell may need to be repeated at intervals as frequent as every few minutes to tens of minutes to ensure sufficient accuracy for robust medical monitoring of a critically ill patient.

In existing RGM devices, the calibration of the optical detection cell is performed by flow switching, in which the respired air flow is diverted away from the sampling cell and the reference gas flow (e.g. nitrogen gas or air) is flowed through the sampling cell. This approach has certain disadvantages as recognized herein. In the case of a reference gas other than air, such as nitrogen, the reference gas must be available in the patient's hospital room and connected with the RGM device. If air is used as the reference gas, then there is potential for error due to residual levels of the target gas (e.g. CO₂ in the case of a capnometer) which may be present in the air.

Another disadvantage recognized herein is that the respiration gas monitoring is interrupted for a time interval as the flow through the sampling chamber of the RGM device is switched from the respiration gas flow to the reference gas flow, and then is switched back from the reference gas flow to the respiration gas flow. Besides the apparent interruption of monitoring data, there is the potential for generating erroneous measurements if the respiratory gas monitoring measurements are resumed before the respiration gas flow through the sampling cell is reinstated and reaches equilibrium.

In view of these difficulties, it is disclosed herein to provide a respiration gas monitor (RGM) device that includes an infrared light source launching infrared light through a respired air flow path, and an optical detector that detects the infrared light after passing through the respired air flow path. An absorption line bandpass filter has a passband encompassing an absorption line of a target gas. A reference line bandpass filter has a passband over which the respired air is transparent. A control device switches the RGM device between: a monitoring state in which the absorption line bandpass filter is in the path of the infrared light; and a calibration state in which the reference line bandpass filter is in the path of the infrared light and the absorption line bandpass filter is not in the path of the infrared light. In this way, the calibration can be performed rapidly, without requiring any interruption of the flow of the respired air through the respired air flow path.

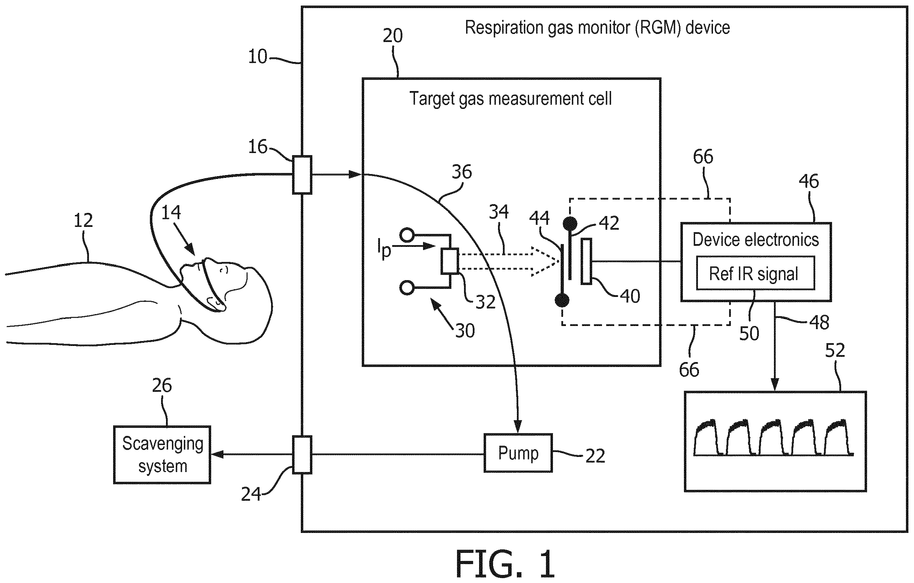

With reference to FIGURE 1, an illustrative respiration gas monitor (RGM) device **10** is connected with a patient **12** by a suitable patient accessory, such as a nasal cannula **14** in the illustrative example, or by an airway adaptor connecting with an endotracheal tube used for mechanical ventilation, or so forth. The patient accessory **14** may optionally include one or more ancillary components, such as an air filter, water trap, or the like (not shown). In the illustrative RGM device **10,** respired air is drawn from the patient accessory **14** into an air inlet **16** and through target gas measurement cell **20** by a pump **22.** The respired air is then discharged via an air outlet **24** of the RGM device **10** to atmosphere or, as in the illustrative embodiment, is discharged through the air outlet **24** into a scavenging system **26** to remove an inhaled anesthetic or other inhaled medicinal agent before discharge into the atmosphere. It should be noted that the respired air generally has a composition that is different from the ambient air, for example the respired air may contain different concentrations of CO₂, oxygen, and/or may contain added gas such as an administered anesthetic gas.

The illustrative RGM device setup has a sidestream configuration in which respired air is drawn into the RGM device using the pump **22,** and the target gas measurement cell **20** is located inside the RGM device **10.** The sidestream configuration is suitably used for a spontaneously breathing patient, i.e. a patient who is breathing on his or her own without assistance of a mechanical ventilator. In an alternative configuration, known as a mainstream configuration (not illustrated), the target gas measurement cell is located externally from the RGM device housing, typically as a target gas measurement cell patient accessory that is inserted into the "mainstream" airway flow of the patient. Such a mainstream configuration may, for example, be employed in conjunction with a mechanically ventilated patient, with the target gas measurement cell patient accessory being designed to mate into an accessory receptacle of the ventilator unit, or is installed on an airway hose feeding into the ventilator.

The target gas measurement cell **20** comprises an infrared optical absorption cell in which the target gas in the respired air drawn from the patient accessory **14** produces absorption in the infrared that is detected optically. By way of non-limiting illustration, CO₂ has an absorption peak at about 4.3 micron. By way of further non-limiting illustration, other target gases may include oxygen (O₂), nitrous oxide (N₂O), or an administered anesthetic gas, each of which have specific characteristic absorption lines in the infrared. An infrared light source **30** includes an infrared emitting element **32** that is resistively heated by a drive current I_{P} which in some embodiments comprises an electric current pulse train. The electric current I_{P} heats the infrared emitting element (e.g. a ceramic element or other thermally radiating element) to emit blackbody radiation with strong emission in the infrared. Thusly launched broadband infrared light **34** (diagrammatically indicated by a block arrow in FIGURE 1) transmits through a respired air flow path **36** (diagrammatically indicated by a curve arrow in FIGURE 1) along which the respired air flows. The flow path **36** may be defined by a tube or other conduit defining a cuvette with walls made of a plastic, glass, sapphire, or other material that is substantially transparent for the infrared light **34.** The pump **22** actively drives the flow of respired air through the flow path **36;** in a mainstream configuration the flow may be driven by mechanical ventilation of the patient, and/or by active breathing of the patient.

An optical detector **40** is configured to detect the infrared light **34.** In some illustrative embodiments, the optical detector **40** may be a lead selenide (PbSe) detector, a microbolometer, thermocouple, pyroelectric detector, or the like. To provide specificity to the target gas, an absorption line bandpass filter **42** having a passband tuned to an absorption line of the target gas is interposed between the infrared emitting element **32** and the optical detector **40.** For example, in the case of the target gas being CO₂, the absorption line bandpass filter **42** suitably has a passband that encompasses, and is preferably centered at, 4.3 micron which is a wavelength at which carbon dioxide is strongly absorbing. For other target gases, the bandpass filter is designed to have a passband that encompasses, and preferably is centered on, a strong absorption line of the other target gas. The absorption line bandpass filter may, for example, comprise a stack of layers on an infrared light-transmissive substrate such as sapphire, in which the layers of the stack of layers have thicknesses, refractive indices, and arrangement designed to form an interference filter with a narrow passband having the requisite center frequency (e.g. 4.3 micron for CO₂ detection).

For performing the calibration, the illustrative embodiment of FIGURE 1 employs a separate reference line bandpass filter **44** constructed similarly to the absorption line bandpass filter **42**, but with the stack of layers tuned to a wavelength at which no component present in non-negligible quantities in the respired air exhibits strong absorption. In some embodiments, the reference line bandpass filter **44** has a narrow passband that encompasses, and preferably is centered on, a frequency of 3.6 micron, although other wavelengths for which the respired air is transparent are also contemplated. In general, the reference line bandpass filter **44** has a passband over which the respired air carried in the respired air flow path **36** is transparent. By "transparent" it is meant that absorption by the respired air is negligibly small over the passband of the reference line bandpass filter **44** when compared with absorption by the target gas over the passband of the absorption line bandpass filter **42** for the lowest concentration or partial pressure of the target gas in the respired air that the RGM device **10** is designed to detect.

The RGM device **10** further includes RGM device electronics **46** that provide electrical biasing of, and readout for, the optical detector **40.** The electronics **46** optionally provide the drive current I_{P} for the infrared light source **30** (connection not shown in FIGURE 1; moreover other driving configurations are contemplated such as a separate current or voltage drive power supply). The RGM device electronics **46** also include analog signal processing circuitry and/or digital signal processing (DSP) suitable for converting the detected signal into a gas signal **48**, e.g. a concentration or partial pressure of CO₂ in the respired air flow, and optionally for performing further processing such as detecting a breath interval and/or an end-tidal CO₂ level (etCO₂ level, relevant for capnometry embodiments). The conversion to CO₂ level or other target gas signal can employ suitable empirical calibration - for example, in general, concentration or partial pressure of the target gas produces greater absorption and hence a reduced signal from the optical detector **40.** The empirical calibration may take into account other factors such as flow rate or pressure, and/or the effects of gases other than the target gas (for example, oxygen and nitrous oxide are known to affect the infrared absorption characteristics due to CO₂), and can be suitably programmed as a look-up table, mathematical equation, non-linear op-amp circuit, or so forth.

Another aspect of the conversion is compensating for drift in the signal output by the optical detector **40.** Such drift may be due to drift in the detector **40**, and/or due to drift in the intensity of the infrared radiation **34** launched by the infrared light source **30**, and/or due to other factors such as condensate buildup on walls of the path **36** through which the respired air flows. To account for such drift, a calibration is occasionally performed using the reference line bandpass filter **44** as described elsewhere herein in order to generate a reference infrared (IR) signal **50.** By employing a ratio of the signal from the optical detector **40** versus the reference IR signal **50**, such drift is compensated.

In the case of the RGM device electronics **46** being implemented at least in part by DSP, such DSP may be implemented by a microcontroller or microprocessor or the like programmed by instructions stored on a read only memory (ROM), electronically programmable read-only memory (EPROM), CMOS memory, flash memory, or other electronic, magnetic, optical or other non-transitory storage medium that is readable and executable by the microcontroller or microprocessor or the like to perform the digital signal processing. For DSP processing, a front-end analog-to-digital (A/D) conversion circuit is typically provided to digitize the detector signal from the optical detector **40.** To provide useful target gas monitoring, an output component **52** is provided. In the illustrative embodiment, the output component **52** is a display **52,** e.g. an LCD display or the like. The illustrative display **52** plots target gas concentration or partial pressure versus time as a trend line. Additionally or alternatively, the display may show a numerical value, e.g. of the target gas concentration at a particular time in the respiratory cycle, e.g. etCO₂ in the case of a capnometer. The output component may additionally or alternatively take other forms, such as being or including (possibly in addition to the display **52**) a USB port or other data port via which the target gas data may be read out.

It will be further appreciated that the RGM device **10** may include numerous other components not illustrated in simplified diagrammatic FIGURE 1, such as a pressure gauge and/or flow meter for monitoring the respired air flow, a keypad or other user input components, and/or so forth.

FIGURE 1 illustrates both the absorption line bandpass filter **42** and the reference line bandpass filter **44** in the path of the infrared light **34.** In actual operation, however, a control device is operative to switch the RGM device **10** between a monitoring state and a calibration state. In the monitoring state, the absorption line bandpass filter **42** is in the path of the infrared light **34** and the reference line bandpass filter **44** is not in the path of the infrared light **34.** The monitoring state is the normal operating state, and provides for the optical detector **40** to measure the absorption of the infrared light due to the target gas (e.g. CO₂), since the absorption line bandpass filter **42** encompasses (and is preferably centered on) an absorption line of the target gas (e.g. 4.3 micron in the case of CO₂target gas). When switched to the calibration state, the reference line bandpass filter **44** is in the path of the infrared light **34** and the absorption line bandpass filter **42** is not in the path of the infrared light **34.** This state allows for measurement of the reference infrared signal **50,** since the reference line bandpass filter **44** is chosen to have a passband over which the respired air is transparent (e.g., 3.6 micron in illustrative examples herein). In this way, the reference infrared signal **50** can be measured without changing out the gas flow from respired gas to air or nitrogen. In the following, some illustrative embodiments of the control device for switching between the monitoring state and the calibration state are described.

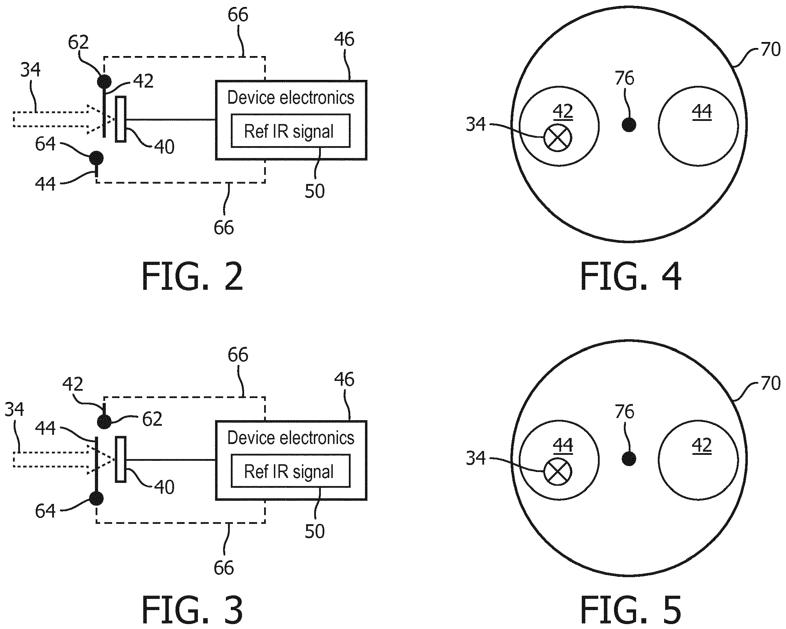

With reference to FIGURES 2 and 3, in one illustrative embodiment the control device comprises a filter flipper comprising a first filter flipper **62** that mechanically moves the absorption line bandpass filter **42** into or out of the path of the infrared light **34,** and a second filter flipper **64** that mechanically moves the reference line bandpass filter **44** into or out of the path of the infrared light **34.** The two filter flippers **62, 64** are coupled together, e.g. mechanically or by control logic or electrical control circuitry **66** (e.g., connected with or part of the device electronics **46** as diagrammatically indicated in FIGURES 1-3), so as to switch to the monitoring state by the first filter flipper **62** mechanically moving the absorption line bandpass filter **42** into the path of the infrared light **34** and the second filter flipper **64** mechanically moving the reference line bandpass filter **44** outside of the path of the infrared light **34,** as shown in FIGURE 2. The two filter flippers **62, 64** are similarly switchable to the calibration state by the second filter flipper **64** mechanically moving the reference line bandpass filter **44** into the path of the infrared light **34** and the first filter flipper **62** mechanically moving the absorption line bandpass filter **44** outside of the path of the infrared light. In some embodiments, the switching between the monitoring state and the calibration state is fast, e.g. 0.2 sec in some contemplated embodiments. In some embodiments the RGM **10** switches from the monitoring state (FIGURE 2) to the calibration state (FIGURE 3), measures the reference infrared signal **50,** and switches back to the monitoring state (FIGURE 2) sufficiently quickly compared with a single respiration cycle (e.g. 10-20 sec for a respiration rate of 3-6 breaths per minute) so that the sampling of the target gas waveform is advantageously not significantly disturbed by the calibration.

The design of the filter flipper assembly **62, 64** can take various forms. In one embodiment each bandpass filter **42, 44** is mounted on a rotating axis and the two filter flippers **62, 64** are motors that rotate the respective filters **42, 44** about those axes into and out of the path of the infrared light **34,** as diagrammatically shown in FIGURES 2 and 3.

With reference to FIGURES 4 and 5, in another illustrative embodiment the filter flipper comprises a filter wheel **70** that rotates about an axis **76,** and on which both the absorption line bandpass filter **42** and the reference line bandpass filter **44** are mounted. FIGURES 4 and 5 illustrate the monitoring state (FIGURE 4) and the calibration state (FIGURE 5), respectively, viewed along the optical axis of the infrared light **34.** In the monitoring state shown in FIGURE 4, the filter wheel **70** is rotated about its axis **76** such that the absorption line bandpass filter **42** is rotated into the infrared light **34.** In the calibration state shown in FIGURE 5, the filter wheel **70** is rotated about its axis **76** such that the reference line bandpass filter **44** is rotated into the infrared light **34.** In this design the control device is connected to rotate the filter wheel **70** to implement switching between the monitoring and calibration states.

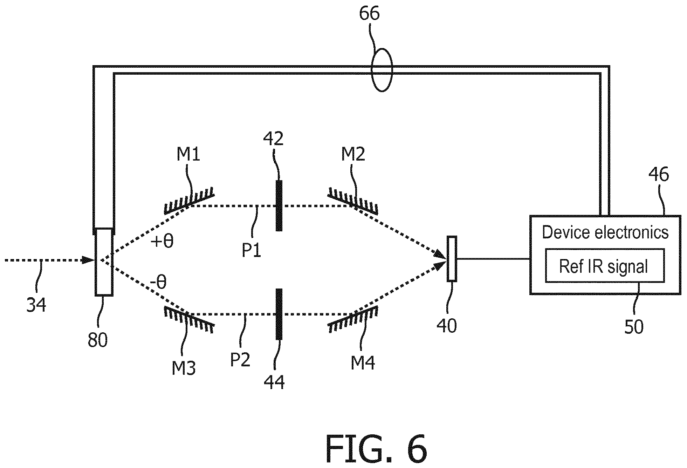

With reference to FIGURE 6, according to the invention, the control device comprises an electro-optical beam steering device **80** operable at a first electric bias or at a second electric bias provided by the electrical control circuitry **66** driven by the device electronics **46.** The optical beam steering device **80** may, for example, employ a liquid crystal device forming a switchable diffraction grating. Such electro-optical beam-steering devices are used, for example, in bar-code scanners to provide fast beam direction switching. When biased at the first electric bias, the illustrative electro-optical beam steering device **80** steers the infrared light **34** at an angle +*θ* to be reflected by mirrors **M1**, **M2** along a first optical path **PI** that passes through the absorption line bandpass filter **42** (and does not pass through the reference line bandpass filter **44**) and then impinges upon the optical detector **40.** When biased at the second electric bias, the illustrative electro-optical beam steering device **80** steers the infrared light **34** at an angle -*θ* to be reflected by mirrors **M3, M4** along a second optical path **P2** that passes through the reference line bandpass filter **44** (and does not pass through the absorption line bandpass filter **42**) and then impinges upon the optical detector **40.**

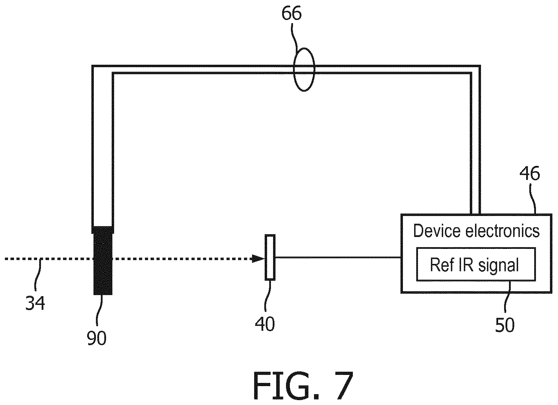

With reference to FIGURE 7, in another illustrative embodiment, the control device comprises an electrically tunable optical bandpass filter **90** operable at a first electric bias or a second electric bias. The electrically tunable optical bandpass filter **90** may, for example, comprise a liquid crystal or acousto-optic device comprising an electrically tunable interference filter that can be switched between two different passbands, e.g. 4.3 micron in the illustrative CO₂ target gas example, and 3.6 micron as the reference passband. The electrically tunable optical bandpass filter **90** is always disposed in the path of the infrared light **34,** but can be tuned to instantiate either the absorption line bandpass filter **42** or the reference line bandpass filter **44.** More particularly, when biased at the first electric bias, the monitoring state is implemented by tuning the electrically tunable optical bandpass filter **90** to the passband of the absorption line bandpass filter **42,** whereby the electrically tunable optical bandpass filter **90** instantiates the absorption line bandpass filter **42.** When biased at the second electric bias, the calibration state is implemented by tuning the electrically tunable optical bandpass filter **90** to the passband of the reference line bandpass filter **44,** whereby the electrically tunable optical bandpass filter **90** instantiates the reference line bandpass filter **44.**

The control device embodiments of FIGURES 2-5 and 7 are merely illustrative examples, and other configurations are contemplated. Advantageously, the control device **62, 64, 70, 80, 90** is not operative to divert flow of respired air through the respired air flow path **36** when operating to switch the RGM device **10** to the calibration state. Rather, the control device switches between the monitoring state and the calibration state, and indeed performs the calibration, without the need to interrupt or alter flow of respired air through the respired air flow path **36.** This promotes stability of the respired air flow and eliminates transient periods when the respired air flow may not yet be stabilized after a calibration operation, and thereby improves accuracy of the RGM device **10.**

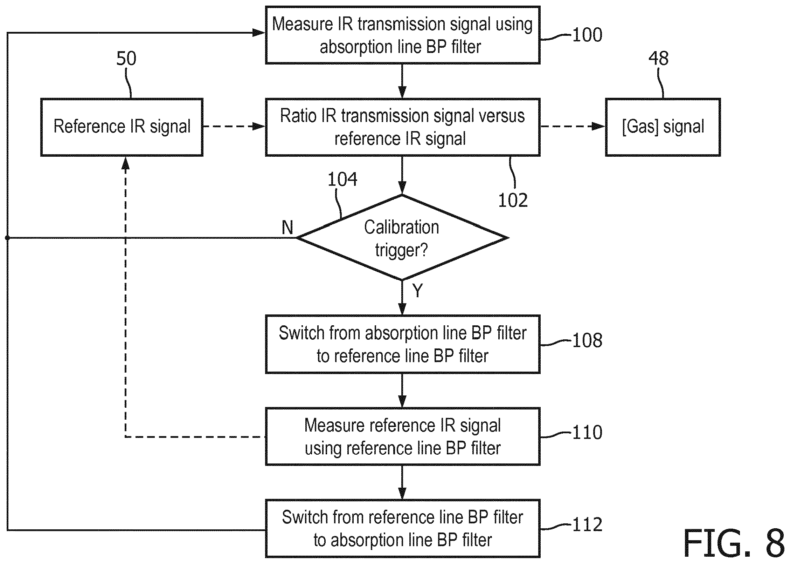

With reference to FIGURE 8, operation of the RGM device **10** of FIGURE 1 is diagrammatically flowcharted. The monitoring state is depicted by operation **100** in which the infrared (IR) transmission signal is measured using the absorption line bandpass (BP) filter **42** and an operation **102** in which a ratio of the IR transmission signal and the reference IR signal **50** is computed to generate the target gas concentration or partial pressure signal **48.** As previously noted, the signal processing operation **102** may optionally account other factors such as flow rate or pressure, and/or the effects of gases other than the target gas (e.g. oxygen and/or nitrous oxide which can affect the infrared absorption characteristics due to CO₂), and these can be suitably programmed as a look-up table, mathematical equation, non-linear op-amp circuit, or so forth.

At a decision operation **104,** it is determined whether the RGM device **10** should switch from the monitoring state to the calibration state in order to update the value of the reference IR signal **50.** The decision **104** can be based on various chosen factors. In one embodiment, the decision **104** switches to the calibration state after a fixed time interval, e.g. after every 5 minutes of monitoring. In another embodiment, the decision **104** switches when the target gas signal **48** drifts over time by more than some threshold amount, in order to ensure that the drift is not due to a change in the reference signal (e.g. due to drift of the intensity of the infrared light **34** output by the infrared light source **30,** or due to drift of the optical detector **40,** or due to contamination of the walls of the respired air flow path **36,** or so forth). So long as the decision operation **104** does not call for updating the calibration, flow returns to the monitoring state IR transmission measurement operation **100** to continue monitoring the target gas.

On the other hand, if the decision operation **104** calls for a calibration update, then in an operation **108** the RGM device **10** switches from the monitoring state to the calibration state by switching from the absorption line bandpass filter **42** to the reference line bandpass filter **44,** e.g. using any one of the control devices described with reference to FIGURES 2-7. Now in the calibration state, a measurement operation **110** is performed which is analogous to the measurement operation **100** except now with the infrared light being filtered by the reference bandpass filter. This measurement **110** then provides the updated value for the reference infrared signal **50.** In an operation **112,** the RGM device **10** switches from the calibration state back to the monitoring state by switching from the reference line bandpass filter **44** back to the absorption line bandpass filter **42.** Flow then returns to operation **100** to resume monitoring of the target gas. Advantageously, the calibration process sequence **108, 110, 112** involves only a rapid change-out of the bandpass filters **42, 44,** e.g. by a mechanical filter flipper (e.g. illustrative embodiments of FIGURES 2-5), fast electro-optical beam steering (e.g. illustrative embodiment of FIGURE 6), or by using a tunable bandpass optical filter to instantiate the requisite filter (e.g. illustrative embodiment of FIGURE 7). Thus, the calibration can be performed rapidly so as to introduce negligible interruption in the [CO₂] waveform or other target gas waveform generated by the monitoring state. This in turn facilitates more frequent updating of the reference infrared signal **50** and consequently more accurate monitoring of the target gas.

The invention has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

## Claims

1. A respiration gas monitor (RGM) device comprising:
a respired air flow path (36) for carrying respired air;
an infrared light source (30) arranged to launch infrared light (34) through the respired air flow path;
an optical detector (40) arranged to detect the infrared light after passing through the respired air flow path;
an absorption line bandpass filter (42) having a passband that encompasses an absorption line of a target gas;
a reference line bandpass filter (44) having a passband over which the respired air is transparent;
a control device (62, 64, 70, 80, 90) operative to switch the RGM device between:
a monitoring state in which the absorption line bandpass filter is in the path of the infrared light and the reference line bandpass filter is not in the path of the infrared light, and
a calibration state in which the reference line bandpass filter is in the path of the infrared light and the absorption line bandpass filter is not in the path of the infrared light; and **characterised by**
an electro-optical beam steering device (80) operable at:
a first electric bias implementing the monitoring state by steering the infrared light to an optical path (P1) that passes through the absorption line bandpass filter and does not pass through the reference line bandpass filter, and
a second electric bias implementing the calibration state by steering the infrared light to an optical path (P2) that passes through the reference line bandpass filter and does not pass through the absorption line bandpass filter.

2. The RGM device of claim 1 wherein the control device comprises:
a filter flipper (62, 64, 70) connected to:
switch to the monitoring state by mechanically moving the absorption line bandpass filter into the path of the infrared light and mechanically moving the reference line bandpass filter outside of the path of the infrared light, and
switch to the calibration state by mechanically moving the reference line bandpass filter into the path of the infrared light and mechanically moving the absorption line bandpass filter outside of the path of the infrared light.

3. The RGM device of claim 2 wherein the filter flipper comprises a filter wheel (70).

4. The RGM device of claim 1 wherein the control device comprises:
an electrically tunable optical bandpass filter (90) operable at:
a first electric bias implementing the monitoring state by tuning the electrically optical tunable bandpass filter to the passband of the absorption line bandpass filter whereby the electrically tunable optical bandpass filter instantiates the absorption line bandpass filter (42); and
a second electric bias implementing the calibration state by tuning the electrically tunable optical bandpass filter to the passband of the reference line bandpass filter whereby the electrically tunable optical bandpass filter instantiates the reference line bandpass filter (44).

5. The RGM device of any one of claims 1-4 wherein the reference line bandpass filter (44) has a passband encompassing 3.6 micron.

6. The RGM device of any one of claims 1-5 wherein the target gas is carbon dioxide and the absorption line bandpass filter (44) has a passband encompassing the 4.3 micron absorption line of carbon dioxide.

7. The RGM device of any one of claims 1-6 further comprising:
electronics (46) configured to:
measure a reference infrared signal (50) using the optical detector (40) with the RGM device in the calibration state, and to
measure a concentration or partial pressure of the target gas in the respired air using the optical detector with the RGM device in the monitoring state and further using the reference infrared signal.

8. The RGM device of any one of claims 1-7 wherein the control device is configured to switch the RGM device to the calibration state without interrupting or altering flow of respired air through the respired air flow path.

9. A method of operating a respiration gas monitor (RGM) device, the method comprising:
flowing respired air through a respired air flow path (36);
launching infrared light (34) through the respired air flow path;
while flowing the respired air through the respired air flow path, performing target gas monitoring including measuring an infrared transmission signal indicating transmission of the launched infrared light through the respired air flow path with an absorption line bandpass filter (42) disposed in the path of the infrared light and determining a value for the target gas in the respired air from the infrared transmission signal and a reference infrared signal;
while flowing the respired air through the respired air flow path, performing a calibration including measuring the reference infrared signal indicating transmission of the launched infrared light through the respired air flow path with a reference line bandpass filter (44) disposed in the path of the infrared light; **characterised by**
performing the target gas monitoring by operating an electro-optic beam steering device (80) to steer the infrared light to an optical path (P1) that passes through the absorption line bandpass filter and does not pass through the reference line bandpass filter; and
performing the calibration by operating the electro-optic beam steering device (80) to steer the infrared light to an optical path (P2) that passes through the reference line bandpass filter and does not pass through the absorption line bandpass filter.

10. The method of claim 9 wherein:
the target gas monitoring is performed with the RGM device in a monitoring state in which the absorption line bandpass filter is disposed in the path of the infrared light and the reference line bandpass filter is disposed outside of the path of the infrared light, and
the calibration is performed with the RGM device in a calibration state in which the reference line bandpass filter is disposed in the path of the infrared light and the absorption line bandpass filter is disposed outside of the path of the infrared light.

11. The method of claim 10 further comprising:
moving the absorption line bandpass filter and the reference line bandpass filter using a filter flipper (62, 64, 70) to switch between monitoring state and the calibration state.

12. The method of claim 9 wherein:
the target gas monitoring is performed with the RGM device in a monitoring state in which an electrically tunable optical bandpass filter (90) instantiates the absorption line bandpass filter (42) by being tuned to a passband that encompasses an absorption line of a target gas; and
the calibration is performed with the RGM device in a calibration state in which the electrically tunable optical bandpass filter instantiates the reference line bandpass filter (44) by being tuned to a passband over which the respired air is transparent.

13. The method of any one of claims 9-12 wherein the absorption line bandpass filter (42) has a passband that encompasses an absorption line of a target gas and the reference line bandpass filter (44) has a passband over which the respired air is transparent.

## Patentansprüche

1. Respirationsgasüberwachungs(RGM)-Vorrichtung, umfassend:
einen Atemluftströmungsweg (36), in dem Atemluft transportiert wird;
eine Infrarotlichtquelle (30), die dafür eingerichtet ist, Infrarotlicht (34) durch den Atemluftströmungsweg zu schicken;
einen optischen Detektor (40), der dafür eingerichtet ist, das Infrarotlicht zu erfassen, das den Atemluftströmungsweg passiert;
ein Absorptionslinien-Bandpassfilter (42), das einen Durchlassbereich aufweist, der eine Absorptionslinie eines Taget-Gases umfasst;
ein Bezugslinien-Bandpassfilter (44), das einen Durchlassbereich aufweist, über dem die Atemluft transparent ist;
eine Steuervorrichtung (62, 64, 70, 80, 90), die betreibbar ist, um die RGM-Vorrichtung umzuschalten zwischen:
einem Überwachungszustand, in dem das Absorptionslinien-Bandpassfilter im Weg des Infrarotlichts liegt und das Bezugslinien-Bandpassfilter nicht im Weg des Infrarotlichts liegt, und
einem Kalibrierungszustand, in dem das Bezugslinien-Bandpassfilter im Weg des Infrarotlichts liegt, und
das Absorptionslinien-Bandpassfilter nicht im Weg des Infrarotlichts liegt;
und **gekennzeichnet durch**
eine elektro-optische Strahllenkungsvorrichtung (80), die betreibbar ist bei:
einer ersten elektrischen Vorspannung, die durch Lenken des Infrarotlichts auf einen optischen Weg (P1), der durch das Absorptionslinien-Bandpassfilter verläuft und nicht durch das Bezugslinien-Bandpassfilter verläuft, den Überwachungszustand implementiert, und
einer zweiten elektrischen Vorspannung, die durch Lenken des Infrarotlichts auf einen optischen Weg (P2), der durch das Bezugslinien-Bandpassfilter verläuft und nicht durch das Absorptionslinien-Bandpassfilter verläuft, den Kalibrierungszustand implementiert.

2. RGM-Vorrichtung nach Anspruch 1 wobei die Steuervorrichtung umfasst:
einen Filterflipper (62, 64, 70), der verbunden ist zum:
Umschalten auf den Überwachungszustand durch mechanisches Bewegen des Absorptionslinien-Bandpassfilters in den Weg des Infrarotlichts und mechanisches Bewegen des Bezugslinien-Bandpassfilters aus dem Weg des Infrarotlichts, und
Umschalten auf den Kalibrierungszustand durch mechanisches Bewegen des Bezugslinien-Bandpassfilters in den Weg des Infrarotlichts und mechanisches Bewegen des Absorptionslinien-Bandpassfilters aus dem Weg des Infrarotlichts.

3. RGM-Vorrichtung nach Anspruch 2 wobei der Filterflipper ein Filterrad (70) umfasst.

4. RGM-Vorrichtung nach Anspruch 1, wobei die Steuervorrichtung umfasst:
ein elektrisch abstimmbares optisches Bandpassfilter (90), das betätigbar ist bei:
einer ersten elektrischen Vorspannung, die den Überwachungszustand implementiert durch Abstimmen des elektrisch abstimmbaren optischen Bandpassfilters auf den Durchlassbereich des Absorptionslinien-Bandpassfilters, wodurch das elektrisch abstimmbare optische Bandpassfilter das Absorptionslinien-Bandpassfilter (42) instanziiert; und
einer zweiten elektrischen Vorspannung, die den Kalibrierungszustand implementiert durch Abstimmen des elektrisch abstimmbaren optischen Bandpassfilters auf den Durchlassbereich des Bezugslinien-Bandpassfilters,
wodurch das elektrisch abstimmbare optische Bandpassfilter das Bezugslinien-Bandpassfilter (44) instanziiert.

5. RGM-Vorrichtung nach einem der Ansprüche 1 bis 4, wobei das Bezugslinien-Bandpassfilter (44) einen Durchlassbereich aufweist, der 3,6 Mikrometer umfasst.

6. RGM-Vorrichtung nach einem der Ansprüche 1 bis 5, wobei das Target-Gas Kohlendioxid ist und das Absorptionslinien-Bandpassfilter (44) einen Durchlassbereich aufweist, der die 4,3 Mikrometer-Absorptionslinie von Kohlendioxid umfasst.

7. RGM-Vorrichtung nach einem der Ansprüche 1 bis 6, ferner umfassend:
Elektronik (46), die ausgelegt ist zum:
Messen eines Bezugs-Infrarotsignals (50) unter Verwendung des optischen Detektors (40), wenn die RGM-Vorrichtung im Kalibrierungszustand ist, und
Messen einer Konzentration oder eines Partialdrucks des Target-Gases in der Atemluft unter Verwendung des optischen Detektors, wenn die RGM-Vorrichtung im Überwachungszustand ist, und ferner unter Verwendung des Bezugs-Infrarotsignals.

8. RGM-Vorrichtung nach einem der Ansprüche 1 bis 7, wobei die Steuervorrichtung dafür ausgelegt ist, die RGM-Vorrichtung auf den Kalibrierungszustand umzuschalten, ohne einen Strom von Atemluft durch den Atemluftströmungsweg zu unterbrechen oder zu verändern.

9. Verfahren zum Betreiben einer Respirationsgasüberwachungs(RGM)-Vorrichtung, wobei das Verfahren umfasst:
Strömen lassen von Atemluft durch einen Atemluftströmungsweg (36);
Schicken von Infrarotlicht (34) durch den Atemluftströmungsweg;
Durchführen einer Überwachung eines Target-Gases, während die Atemluft durch den Atemluftströmungsweg strömt, einschließlich eines Messens eines Infrarotsendesignals, das eine Übertragung des abgeschickten Infrarotlichts durch den Atemluftströmungsweg anzeigt, während ein Absorptionslinien-Bandpassfilter (42) im Weg des Infrarotlichts angeordnet ist, und Bestimmen eines Wertes für das Target-Gas in der Atemluft aus dem Infrarotsendesignal und einem Bezugs-Infrarotsignal;
Durchführen einer Kalibrierung, während die Atemluft durch den Atemluftströmungsweg strömt, einschließlich eines Messens des Bezugs-Infrarotsignals, das eine Übertragung des abgeschickten Infrarotlichts durch den Atemluftströmungsweg anzeigt, während ein Bezugslinien-Bandpassfilter (44) in dem Weg des Infrarotlichts angeordnet ist; **gekennzeichnet durch**:
Durchführen der Überwachung des Target-Gases durch Betätigen einer elektro-optischen Strahllenkungsvorrichtung (80), um das Infrarotlicht auf einen optischen Weg (P1) zu lenken, der durch das Absorptionslinien-Bandpassfilter verläuft und nicht durch das Bezugslinien-Bandpassfilter verläuft; und
Durchführen der Kalibrierung durch Betätigen der elektro-optischen Strahllenkungsvorrichtung (80), um das Infrarotlicht auf einen optischen Weg (P2) zu lenken, der durch das Bezugslinien-Bandpassfilter verläuft und nicht durch das Absorptionslinien-Bandpassfilter verläuft.

10. Verfahren nach Anspruch 9, wobei:
die Überwachung des Target-Gases durchgeführt wird, wenn die RGM-Vorrichtung in einem Überwachungszustand ist, in dem das Absorptionslinien-Bandpassfilter im Weg des Infrarotlichts liegt und das Bezugslinien-Bandpassfilter nicht im Weg des Infrarotlichts liegt, und
die Kalibrierung durchgeführt wird, wenn die RGM-Vorrichtung in einem Kalibrierungszustand ist, in dem das Bezugslinien-Bandpassfilter im Weg des Infrarotlichts liegt und das Absorptionslinien-Bandpassfilter nicht im Weg des Infrarotlichts liegt.

11. Verfahren nach Anspruch 10, ferner umfassend:
Bewegen des Absorptionslinien-Bandpassfilters und des Bezugslinien-Bandpassfilters unter Verwendung des Filterflippers (62, 64, 70), um zwischen einem Überwachungszustand und dem Kalibrierungszustand umzuschalten.

12. Verfahren nach Anspruch 9, wobei:
die Überwachung des Target-Gases durchgeführt wird, während die RGM-Vorrichtung in einem Überwachungszustand ist, in dem ein elektrisch abstimmbares Bandpassfilter (90) das Absorptionslinien-Bandpassfilter (42) instanziiert, weil es auf einen Durchlassbereich abgestimmt wird, der eine Absorptionslinie eines Target-Gases umfasst; und
die Kalibrierung durchgeführt wird, während die RGM-Vorrichtung in einem Kalibrierungszustand ist, in dem das elektrisch abstimmbare Bandpassfilter das Bezugslinien-Bandpassfilter (44) instanziiert, weil es auf einen Durchlassbereich abgestimmt wird, über dem die Atemluft transparent ist.

13. Verfahren nach einem der Ansprüche 9 bis 12, wobei das Absorptionslinien-Bandpassfilter (42) einen Durchlassbereich aufweist, der eine Absorptionslinie eines Target-Gases umfasst, und das Bezugslinien-Bandpassfilter (44) einen Durchlassbereich aufweist, über dem die Atemluft transparent ist.

## Revendications

1. Dispositif de surveillance du gaz respiratoire (RGM) comprenant :
un trajet d'écoulement d'air respiré (36) destiné au transport de l'air respiré ;
une source lumineuse infrarouge (30) conçue pour lancer une lumière infrarouge (34) à travers le trajet d'écoulement d'air respiré ;
un détecteur optique (40) conçu pour détecter la lumière infrarouge après avoir traversé le trajet d'écoulement d'air respiré ;
un filtre passe-bande de raie d'absorption (42) présentant une bande passante, laquelle englobe une raie d'absorption d'un gaz cible ;
un filtre passe-bande de raie de référence (44) présentant une bande passante sur laquelle l'air respiré est transparent ;
un dispositif de commande (62, 64, 70, 80, 90) utilisable pour commuter le dispositif RGM entre :
un état de surveillance dans lequel le filtre passe-bande de raie d'absorption se trouve sur le trajet de la lumière infrarouge et le filtre passe-bande de raie de référence ne se trouve pas sur le trajet de la lumière infrarouge, et
un état d'étalonnage dans lequel le filtre passe-bande de raie de référence se trouve sur le trajet de la lumière infrarouge et le filtre passe-bande de raie d'absorption ne se trouve pas sur le trajet de la lumière infrarouge ;
et **caractérisé par**
un dispositif d'orientation du faisceau électrooptique (80) utilisable :
à une première polarisation électrique mettant en œuvre l'état de surveillance par direction de la lumière infrarouge vers un trajet optique (P1), lequel passe à travers le filtre passe-bande de raie d'absorption et ne passe pas à travers le filtre passe-bande de raie de référence, et
à une seconde polarisation électrique mettant en œuvre l'état d'étalonnage par direction de la lumière infrarouge vers un trajet optique (P2), lequel traverse le filtre passe-bande de raie de référence et ne traverse pas le filtre passe-bande de raie d'absorption.

2. Dispositif RGM selon la revendication 1, dans lequel le dispositif de commande comprend :
une languette de filtre (62, 64, 70) connecté :
pour passer à l'état de surveillance par déplacement mécanique du filtre passe-bande de raie d'absorption dans le trajet de la lumière infrarouge et par déplacement mécanique du filtre passe-bande de raie de référence hors du trajet de la lumière infrarouge, et
pour passer à l'état d'étalonnage par déplacement mécanique du filtre passe-bande de raie de référence dans le trajet de la lumière infrarouge et par déplacement mécanique du filtre passe-bande de raie d'absorption hors du trajet de la lumière infrarouge.

3. Dispositif RGM selon la revendication 2, dans lequel la languette de filtre comprend une roue de filtre (70).

4. Dispositif RGM selon la revendication 1, dans lequel le dispositif de commande comprend :
un filtre passe-bande optique (90) réglable électriquement utilisable :
à une première polarisation électrique mettant en œuvre l'état de surveillance par réglage du filtre passe-bande réglable électriquement sur la bande passante du filtre passe-bande de raie d'absorption, le filtre passe-bande optique réglable électriquement instanciant le filtre passe-bande de raie d'absorption (42) ; et
à une seconde polarisation électrique mettant en œuvre l'état d'étalonnage par réglage du filtre passe-bande optique réglable électriquement à la bande passante du filtre passe-bande de raie de référence, le filtre passe-bande optique réglable électriquement instanciant le filtre passe-bande de raie de référence (44).

5. Dispositif RGM selon l'une quelconque des revendications 1 à 4, dans lequel le filtre passe-bande de raie de référence (44) présente une bande passante englobant 3,6 microns.

6. Dispositif RGM selon l'une quelconque des revendications 1 à 5, dans lequel le gaz cible est le dioxyde de carbone et le filtre passe-bande de raie d'absorption (44) présente une bande passante englobant la raie d'absorption de 4,3 microns du dioxyde de carbone.

7. Dispositif RGM selon l'une quelconque des revendications 1 à 6, comprenant en outre :
une électronique (46) conçue :
pour mesurer un signal infrarouge de référence (50) à l'aide du détecteur optique (40) et du dispositif RGM dans l'état d'étalonnage, et pour
mesurer une concentration ou une pression partielle du gaz cible dans l'air respiré à l'aide du détecteur optique et du dispositif RGM dans l'état de surveillance et à l'aide en outre du signal infrarouge de référence.

8. Dispositif RGM selon l'une quelconque des revendications 1 à 7, dans lequel le dispositif de commande est conçu pour faire passer le dispositif RGM à l'état d'étalonnage sans interrompre ou modifier l'écoulement d'air respiré à travers le trajet d'écoulement d'air respiré.

9. Procédé de fonctionnement d'un dispositif de surveillance du gaz respiratoire (RGM), le procédé comprenant :
la circulation de l'air respiré à travers un trajet d'écoulement d'air respiré (36) ;
le lancement de la lumière infrarouge (34) à travers le trajet d'écoulement d'air respiré ;
la réalisation, tout en faisant circuler l'air respiré à travers le trajet d'écoulement d'air respiré, d'une surveillance du gaz cible comprenant la mesure d'un signal de transmission infrarouge indiquant la transmission de la lumière infrarouge lancée à travers le trajet d'écoulement d'air respiré à l'aide d'un filtre passe-bande de raie d'absorption (42) disposé dans le trajet de la lumière infrarouge et la détermination d'une valeur pour le gaz cible dans l'air respiré à partir du signal de transmission infrarouge et d'un signal infrarouge de référence ;
la réalisation, tout en faisant circuler l'air respiré à travers le trajet d'écoulement d'air respiré, d'un étalonnage comprenant la mesure du signal infrarouge de référence indiquant la transmission de la lumière infrarouge lancée à travers le trajet d'écoulement d'air respiré à l'aide d'un filtre passe-bande de raie de référence (44) disposé dans le trajet de la lumière infrarouge ; **caractérisé par** la réalisation de la surveillance du gaz cible par fonctionnement d'un dispositif d'orientation du faisceau électrooptique (80) pour orienter la lumière infrarouge vers un trajet optique (P1), lequel passe à travers le filtre passe-bande de raie d'absorption et ne passe pas à travers le filtre passe-bande de raie de référence ; et
la réalisation de l'étalonnage par fonctionnement du dispositif d'orientation du faisceau électrooptique (80) pour orienter la lumière infrarouge vers un trajet optique (P2), lequel traverse le filtre passe-bande de raie de référence et ne traverse pas le filtre passe-bande de raie d'absorption.

10. Procédé selon la revendication 9, dans lequel :
la surveillance du gaz cible est réalisée à l'aide du dispositif RGM dans un état de surveillance dans lequel le filtre passe-bande de raie d'absorption est disposé dans le trajet de la lumière infrarouge et le filtre passe-bande de raie de référence est disposé hors du trajet de la lumière infrarouge, et
l'étalonnage est réalisé à l'aide du dispositif RGM dans un état d'étalonnage dans lequel le filtre passe-bande de raie de référence est disposé dans le trajet de la lumière infrarouge et le filtre passe-bande de raie d'absorption est disposé hors du trajet de la lumière infrarouge.

11. Procédé selon la revendication 10, comprenant en outre :
le déplacement du filtre passe-bande de raie d'absorption et du filtre passe-bande de raie de référence à l'aide d'une languette de filtre (62, 64, 70) pour passer entre l'état de surveillance et l'état d'étalonnage.

12. Procédé selon la revendication 9, dans lequel :
la surveillance du gaz cible est réalisée à l'aide du dispositif RGM dans un état de surveillance dans lequel un filtre passe-bande optique réglable électriquement (90) instancie le filtre passe-bande de raie d'absorption (42) en étant réglé sur une bande passante, laquelle englobe une raie d'absorption d'un gaz cible ; et
l'étalonnage est réalisé à l'aide du dispositif RGM dans un état d'étalonnage dans lequel le filtre passe-bande optique réglable électriquement instancie le filtre passe-bande de raie de référence (44) en étant réglé sur une bande passante sur laquelle l'air respiré est transparent.

13. Procédé selon l'une quelconque des revendications 9 à 12, dans lequel le filtre passe-bande de raie d'absorption (42) présente une bande passante, laquelle englobe une raie d'absorption d'un gaz cible et le filtre passe-bande de raie de référence (44) présente une bande passante sur laquelle l'air respiré est transparent.
